(19) Europäisches Patentamt
European Patent Office
Office européen des brevets

(11) Veröffentlichungsnummer: **0 320 452 A2**

# (12) EUROPÄISCHE PATENTANMELDUNG

(21) Anmeldenummer: **88810826.3**

(22) Anmeldetag: **01.12.88**

(51) Int. Cl.4: **C 07 C 51/265**
C 07 C 69/00

(30) Priorität: **09.12.87 CH 4799/87**

(43) Veröffentlichungstag der Anmeldung:
**14.06.89 Patentblatt 89/24**

(84) Benannte Vertragsstaaten:
**BE CH DE ES FR GB IT LI NL**

(71) Anmelder: **CIBA-GEIGY AG**
**Klybeckstrasse 141**
**CH-4002 Basel (CH)**

(72) Erfinder: **Clausen, Martin**
**Birchstrasse 72**
**CH-8057 Zürich (CH)**

**Rys, Paul, Prof. Dr.**
**In der Looren 51**
**CH-8053 Zürich (CH)**

**Wang, Junkuan**
**Habsburgerstrasse 28/44**
**CH-4037 Zürich (CH)**

## (54) Verfahren zur Herstellung von carboxylierten aromatischen Hydroxyverbindungen.

(57) Verfahren zur Herstellung von Verbindungen der Formel HO-Ar-COOH, worin Ar ein gegebenenfalls substituiertes aromatisches Ringsystem ist, indem man Verbindungen der Formel HO-Ar-$R_1$, worin $R_1$ Alkyl mit 1 bis 5 Kohlenstoffatomen ist und Ar die angegebene Bedeutung hat, oder die O-Acylderivate dieser Verbindungen, in einem organischen Lösungsmittel bei 80 bis 130°C und in Gegenwart eines Acylierungsmittels, einer Kobalt- und/oder Manganverbindung und einer Bromidionen liefernden Verbindung mit Sauerstoff oder Sauerstoff abgebenden Verbindungen oxidiert und anschliessend in üblicher Weise hydrolysiert. In Gegenwart der Acylierungsmittel erreicht man ohne Druckanwendung eine selektive Oxidation der Hydroxyaromaten zu carboxylierten Hydroxyverbindungen, die in guter Reinheit und Ausbeute anfallen.
Die verfahrensgemäss erhaltenen Verbindungen sind z.B. wertvolle Zwischenprodukte zur Herstellung von Farbstoffen, Kunststoffen und Pharmazeutika.

EP 0 320 452 A2

**Beschreibung**

## Verfahren zur Herstellung von carboxylierten aromatischen Hydroxyverbindungen

Die vorliegende Erfindung betrifft ein neues, verbessertes Verfahren zur Herstellung von carboxylierten aromatischen Hydroxyverbindungen, insbesondere carboxylierten Phenolen oder Naphtholen, durch selektive katalytische Oxidation der entsprechenden alkylsubstituierten aromatischen Hydroxyverbindungen.

Die Oxidation von Alkylaromaten in Gegenwart von Katalysatoren ist bereits bekannt. So beschreibt z.B. die EP-A-204 119 ein Verfahren zur Herstellung von 2,6-Naphthalindicarbonsäure durch Oxidation von 2,6-Diisopropylnaphthalin in Gegenwart eines Oxidationskatalysators mit den Komponenten Kobalt/Mangan/Alkalimetall/Bromid, wobei die Reaktion in Essig- und/oder Propionsäure unter Druck durchgeführt wird.

Es wurde nun gefunden, dass man ausgehend von alkylsubstituierten aromatischen Hydroxyverbindungen selektiv durch katalytische Oxidation carboxylierte aromatische Hydroxyverbindungen herstellen kann, wenn man die Umsetzung in einem geeigneten Lösungsmittel und in Gegenwart eines Acylierungsmittels durchführt.

Gegenstand der vorliegenden Erfindung ist daher ein Verfahren zur Herstellung von Verbindungen der Formel

(1) HO - Ar - COOH ,

worin Ar ein gegebenenfalls substituiertes aromatisches Ringsystem, vorzugsweise gegebenenfalls substituiertes Phenylen oder Naphthylen ist, das dadurch gekennzeichnet ist, dass man Verbindungen der Formel

(2) RO - Ar - $R_1$ ,

worin R Wasserstoff oder $R_2CO$-, worin $R_2$ Wasserstoff oder $C_1$-$C_4$-Alkyl, $R_1$ Alkyl mit 1 bis 5 Kohlenstoffatomen ist und Ar die angegebene Bedeutung hat, in einem organischen Lösungsmittel bei 80 bis 130°C und in Gegenwart eines Acylierungsmittels, einer Kobalt- und/oder Manganverbindung und einer Bromidionen liefernden Verbindung mit Sauerstoff oder Sauerstoff abgebenden Verbindungen oxidiert und anschliessend in üblicher Weise hydrolysiert.

Weitere Gegenstände der vorliegenden Erfindung sind die verfahrensgemäss hergestellten carboxylierten aromatischen Hydroxyverbindungen sowie deren Verwendung als Zwischenprodukte zur Herstellung von beispielsweise Farbstoffen, Kunststoffen (Polymeren) oder Pharmazeutika, ferner eignen sie sich auch als Farbentwickler in druck- und wärmeempfindlichen Aufzeichnungsmaterialien.

Bei den im erfindungsgemässen Verfahren eingesetzten Verbindungen der Formel (2) handelt es sich um wie angegeben substituierte ein- oder mehrkernige,z.B. zwei- oder dreikernige,Aromaten, vorzugsweise um solche aus der Gruppe der Benzole und Naphthaline.

Die Alkylsubstituenten $R_1$ enthalten 1 bis 5, vorzugsweise 1 bis 3 Kohlenstoffatome und sind beispielsweise Methyl, Aethyl, n-Propyl, iso-Propyl, n-Butyl, iso-Butyl, sek.-Butyl, tert.-Butyl sowie Pentyl und dessen Isomere; bevorzugt sind Methyl, Aethyl und insbesondere Isopropyl.

Die Verbindungen der Formel (2) können gegebenenfalls weitere Substituenten enthalten, wie z.B. Halogen, insbesondere Chlor, Cyano, Nitro, Sulfo und ($C_1$-$C_4$)-Alkoxy.

Vorzugsweise entsprechen die Verbindungen der Formel (2) der Formel HO-Ar-$R_1$, worin Ar und $R_1$ die angegebenen Bedeutungen haben, sowie insbesondere den Formeln

(3) 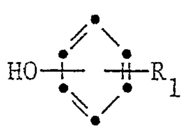 und (4) 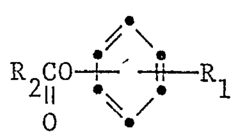 oder

(5) 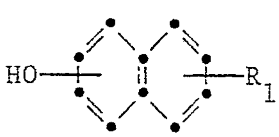 und (6) 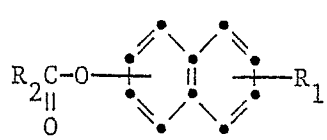 ,

worin $R_1$ die angegebene Bedeutung hat und $R_2$ Wasserstoff, $C_1$-$C_4$-Alkyl, insbesondere $C_1$-$C_3$-Alkyl und vorzugsweise $CH_3$, sowie ferner Phenyl ist.

Bevorzugte Beispiele für die Verbindungen der Formeln (3) und (5) sind p-Methyl- oder p-Isopropylphenol und 2-Methyl-6- oder 2-Isopropyl-6-hydroxynaphthalin und deren $R_2CO$-Derivate, vorzugsweise die Acetylderivate [Formeln (4) und (6), mit $R_1$ Methyl bzw. Isopropyl und $R_2$ Methyl].

Die Verbindungen der Formeln (2) bis (6) sind bekannt und können nach bekannten Verfahren hergestellt werden, z.B. durch Oxidation von entsprechenden Dialkylaromaten und anschliessende übliche Hydrolyse der als Zwischenprodukte gebildeten Monohydroperoxide. Die O-Acylverbindungen kann man dann aus den so erhaltenen Hydroxyverbindungen durch ein übliches Acylierungsverfahren herstellen.

Geeignete Lösungsmittel für die Durchführung des erfindungsgemässen Verfahrens sind solche, die unter den Reaktionsbedingungen stabil sind, vorzugsweise niedermolekulare Alkylcarbonsäuren mit 1 bis 5, vorzugsweise 2 bis 4 Kohlenstoffatomen. Diese werden wie angegeben in Kombination mit einem Acylierungsmittel, wobei es sich vorzugsweise um die Anhydride der genannten organischen Säuren handelt, eingesetzt. Geeignete Säuren sind beispielsweise Ameisen-, Essig-, Propion-, Butter- und Valeriansäure, gegebenenfalls auch die Halogenessigsäuren. Ganz besonders bevorzugt ist indessen die Essigsäure bzw. die Kombination Essigsäure/Acetanhydrid. Gegebenenfalls kann eine solche Kombination auch weitere, unter den Reaktionsbedingungen gegen Sauerstoff beständige Lösungsmittel einschliessen.

Geeignete Oxidationskatalysatoren setzen sich zusammen aus den Oxiden, Hydroxiden, anorganischen oder organischen Salzen des Kobalts und/oder Mangans und aus Bromidionen liefernden Verbindungen, wie Brom, Bromwasserstoffsäure, anorganischen Bromiden oder organischen Bromverbindungen.

Anorganische Kobalt- und/oder Mangansalze sind vorzugsweise die entsprechenden Halogenide, z.B. die Chloride und insbesondere die Bromide, ferner die Carbonate, Sulfate oder Phosphate; als organische Salze kommen solche von aliphatischen Carbonsäuren mit beispielsweise 1 bis 4 Kohlenstoffatomen, wie Formiate, Acetate, Propionate, Lactate, Butyrate, ferner solche von aromatischen Säuren,wie der Benzoe- oder Naphthoesäure,in Betracht. Die Bromide und die Acetate des Kobalts und Mangans sind besonders bevorzugt.

Als Bromidionen liefernde Verbindungen eignen sich anorganische und organische Bromverbindungen, die sich im Reaktionsmedium (Kombination aus einer Säure und einem Säureanhydrid) lösen und Bromidionen zu bilden vermögen. Genannt seien $Br_2$, HBr, die Alkalimetallbromide, wie Lithiumbromid, Natriumbromid oder Kaliumbromid, Schwermetallbromide, wie insbesondere Kobalt- und Manganbromid, ferner Ammoniumbromid, niedere Alkylbromide, wie Methylbromid, Ethylbromid, Bromoform, niedere Alkylenbromide, wie Ethylenbromid, Bromide von niederen Carbonsäuren, wie Bromessigsäure oder Tribromessigsäure.

Bevorzugt sind Bromwasserstoffsäure und die genannten Alkalimetall- und Schwermetallbromide, sowie auch Ammoniumbromid, Ethylbromid und Bromessigsäure.

Werden die Kobalt- und/oder Manganverbindungen (salze) als Bromide eingesetzt, so ist es in der Regel nicht notwendig noch zusätzlich eine sog. Bromidionen liefernde Verbindung einzusetzen.

Die Kobalt- oder Mangansalze werden in der Regel in Mengen von jeweils etwa 2 bis 4 Mol-%, vorzugsweise 2 bis 3 Mol-%, bezogen auf 1 Mol der Verbindungen der Formel (2) eingesetzt.

Verwendet man ein Gemisch aus Kobalt- und Mangansalzen, so beträgt die Menge dieses Gemisches 4 bis 8 Mol-%, vorzugsweise 4 bis 6 Mol-%, bezogen auf 1 Mol der Verbindungen der Formel (2).

Das Gewichtsverhältnis der Salze in der Mischung kann in weiten Grenzen schwanken und beträgt z.B. 1 : 10 bis 10 : 1.

Die Menge der Bromidionen liefernden Verbindungen, die dann eingesetzt werden, wenn die Metallsalze (Kobalt- und Mangansalze), die als Oxidationskatalysatoren dienen, nicht bereits als Bromide vorliegen, kann kleiner oder grösser als die den Metallkationen (Summe aus Kobalt- und Mangankationen) äquivalente Menge sein oder auch dieser Menge entsprechen. Vorzugsweise sollte sie grösser sein als die äquivalente Menge. In der Regel wird daher ein Ueberschuss von etwa 10 bis 20 % über diese äquivalente Menge hinaus eingesetzt.

Gegebenenfalls kann man für die erfindungsgemässe Oxidationsreaktion zusätzlich sog. Reaktionsinitiatoren, wie z.B. organische Radikalbildner aus der Gruppe der Peroxide, vorzugsweise Di-tert.-butyl-peroxid, oder deren Azoverbindungen, z.B. Azobisisobutyronitril, einsetzen.

Die Mengen dieser Initiatoren können etwa 0,1 bis 5,0 Gew.-%, beispielsweise 0,5 bis 5 Gew.-%, bezogen auf das Gewicht der Verbindungen der Formel (2) betragen.

Als eigentliches Oxidationsmittel kann man im erfindungsgemässen Verfahren molekularen Sauerstoff oder auch Sauerstoff enthaltende Gase, wie z.B. Luft, oder Sauerstoff abspaltende Verbindungen, wie z.B. Ozon, verwenden.

Verwendet man Sauerstoff als Oxidationsmittel, so leitet man beispielsweise 0,1 bis 5 $m^3$, vorzugsweise 0,5 bis 2,0 $m^3$, dieses Gases pro kg der Verbindungen der Formel (2) und pro Stunde durch das Reaktionsgemisch.

Die Reaktionszeiten liegen etwa im Bereich von 1 bis 24, vorzugsweise 1 bis 12 und insbesondere bei 4 bis 8 Stunden. Die Reaktionstemperaturen liegen vorzugsweise im Bereich von etwa 90 bis 130° C, insbesondere im Bereich von 110 bis 125° C.

Geht man von den Verbindungen der Formeln (3) bzw. (5) (mit freier Hydroxylgruppe) aus, so kann man das erfindungsgemässe Verfahren so durchführen, dass man diese Verbindungen in einem Acylierungsmittel, insbesondere einem Säureanhydrid, vorzugsweise Acetanhydrid, löst und dann acyliert. Diese Veresterung kann man durch Zusatz einer katalytischen Menge einer Säure, bevorzugt z.B. Bromwasserstoffsäure (48%), und Temperaturerhöhung beschleunigen. In etwa 30 bis 60 Minuten bei 100° C kann vollständige Veresterung erreicht werden.

Sowohl während der anfänglichen Acylierung, vorzugsweise mit einem Säureanhydrid, als auch während der eigentlichen Oxidation, wird Säureanhydrid in die freie Säure übergeführt. Damit ändert sich während des gesamten Oxidationsprozesses das Verhältnis von Säureanhydrid zu Säure.

Es ist daher nicht notwendig, dass die Oxidation ausschliesslich in dem Säureanhydrid als Reaktionsmedium stattfinden muss. Wichtig ist nur, dass immer genügend Säureanhydrid als Acylierungsmittel vorhanden ist, um das Auftreten von freien Hydroxylgruppen während der Oxidation zu vermeiden. Letztere können nämlich die im wesentlichen radikalisch verlaufende Oxidation stoppen.

Die eigentliche Oxidation führt man so durch, dass man als Oxidationsmittel in der Regel Sauerstoff in die Reaktionslösung einleitet und dann gegebenenfalls den Reaktionsinitiator, vorzugsweise Di-tert.-butyl-peroxid oder Azobisisobutyronitril, und anschliessend den Katalysator unter Rühren zugibt. Die Temperatur der Reaktionslösung während der Oxidation wird im angegebenen Bereich, vorzugsweise bei 110 bis 125°C gehalten. Die Oxidation wird durch Abstellen der Sauerstoffzufuhr nach etwa 4 bis 8, beispielsweise nach 6 Stunden beendet.

Die erhaltenen Oxidationsprodukte sind im wesentlichen Verbindungen der Formeln

$$(7) \qquad R_2-\overset{O}{\overset{\|}{C}}-O-Ar-\overset{O}{\overset{\|}{C}}-O-\overset{O}{\overset{\|}{C}}-R_2$$

und

$$(8) \qquad R_2-\overset{O}{\overset{\|}{C}}-O-Ar-\overset{O}{\overset{\|}{C}}-OH \quad ,$$

worin $R_2$ und Ar die angegebenen Bedeutungen haben.

Das Reaktionsgemisch wird dann auf etwa 50 bis 70°C abgekühlt und durch Zugabe von Wasser in einer Menge von z.B. 15 bis 20 Vol-%, bezogen auf die gesamte Lösungsmittelmenge, bei der angegebenen Temperatur während etwa 5 bis 10 Stunden hydrolysiert, wobei die Verbindungen der Formel (7) praktisch quantitativ in die Verbindungen der Formel (8) übergeführt werden.

In der Regel kristallisieren die Verbindungen der Formel (8) aus und werden so aus dem Reaktionsgemisch isoliert, bevor man aus diesen durch weitere Hydrolyse, die sowohl im sauren (Zugabe von z.B. Salz- oder Schwefelsäure) als auch im basischen (Zugabe von z.B. Natriumhydroxidlösung) pH-Wertbereich erfolgen kann, die Verbindungen der Formel (1) erhält; diese werden dann in üblicher Weise, z.B. durch Umkristallisieren, gereinigt.

Die Verbindungen der Formel (7) bleiben in der Regel in Lösung. Diese Lösung, die auch die Katalysatoren bzw. das Katalysatorgemisch enthalten, kann in eine weitere Oxidation eingesetzt werden.

Das erfindungsgemässe Verfahren kann man also diskontinuierlich oder auch kontinuierlich führen. Im letzten Fall muss das Anhydrid/Säure-Verhältnis im Lösungsmittel-Katalysator-Gemisch nach Abtrennen der Verbindungen der Formel (8) für die Wiederverwendung in der Regel entweder durch Zusatz von neuen Anhydrid oder/und durch Abdestillieren von überschüssiger Säure auf den gewünschten Wert eingestellt werden.

Sowohl bei kontinuierlicher als auch bei diskontinuierlicher Reaktionsführung kann auch mit der O-acylierten Verbindung die Reaktion begonnen werden. Die O-Acylierung kann dann vorgängig in einem anderen Reaktionsgefäss durchgeführt werden, und falls vorteilhaft, die O-acylierte Verbindung auch isoliert werden.

Zur Ueberwachung des erfindungsgemässen Verfahrens und zur Analyse der erhaltenen Produkte werden übliche analytische Methoden, insbesondere die Hochdruckflüssigchromatographie (HPLC), verwendet.

Die Vorteile des erfindungsgemässen Verfahrens liegen in erster Linie darin, dass man unter Normaldruck eine selektive Oxidation (Carboxylierung) von Verbindungen der Formel (2) bzw. deren O-Acylderivaten zu Verbindungen der Formel (1) erreichen kann, wenn man in Gegenwart eines Acylierungsmittels arbeitet.

Eine vergleichbare Oxidation in einer Säure (Essigsäure) ist aber unter den erfindungsgemässen Bedingungen (ohne Druck) praktisch nicht durchführbar. Erst bei der Anwendung von Druck wird unter Verwendung von Essigsäure als alleinigem Lösungsmittel eine gewisse Umsetzung erzielt.

Das erfindungsgemässe Oxidationsverfahren liefert die verfahrensgemässen Produkte bei tieferen Reaktionstemperaturen und ohne die Anwendung von Druck in deutlich besserer Reinheit und Ausbeute im Vergleich zu den bekannten Oxidationsverfahren.

Die Verbindungen der Formel (1), gegebenenfalls auch in der Acyloxy-Form [Verbindungen der Formel (8)] sind geeignete Zwischenprodukte für die Herstellung von beispielsweise Farbstoffen, als Comonomere zur Herstellung von Polymeren, die Z.B. zu synthetischen Fasern weiterverarbeitet werden können, oder Pharmazeutika; ferner eignen sie sich z.B. auch als Farbentwickler in druck- und wärmeempfindlichen Aufzeichnungsmaterialien.

Die nachfolgenden Beispiele erläutern das erfindungsgemässe Verfahren, ohne es jedoch auf diese Beispiele zu beschränken. Teile und Prozente beziehen sich, sofern nicht anders angegeben, auf das Gewicht. Die Temperatur ist in Celsiusgraden angegeben.

Beispiel 1

5 g 6-Hydroxy-2-isopropylnaphthalin (Reinheit 99%, HPLC), gelöst in 40 g Essigsäurenahydrid, werden in einem 100 ml-Vierhalskolben, versehen mit Rührer, Thermometer, Rückflusskühler und Gaseinlassfritte gegeben, unter Rühren mit 3 Tropfen Bromwasserstoffsäure (48%) versetzt und dann auf 120°C erhitzt. Nach 30 Minuten Reaktionszeit bei dieser Temperatur beginnt man mit der Sauerstoffzufuhr in einer Menge von 80 ml/min und gibt gleichzeitig 0,1 g Di-tert.-butyl-peroxid und 0,16 g Kobalt(II)acetat-tetrahydrat und 0,2 g

Mangan(II)bromid-tetrahydrat in die Reaktionslösung.

Die Reaktion wird nach 6 Stunden durch Abstellen der Sauerstoffzufuhr beendet. Das Reaktionsgemisch wird auf 60°C abgekühlt, mit 7 ml Wasser versetzt, wobei sich ein pH-Wert im sauren Bereich einstellt, und während 5 bis 10 Stunden bei 60°C hydrolysiert.

Das Reaktionsgemisch gibt man nach Abkühlen auf Raumtemperatur tropfenweise und unter gutem Rühren in 150 ml Eiswasser. Das dabei ausfallende Produkt wird abgetrennt, mit Wasser gewaschen und getrocknet. Ausbeute: 4,6 g 6-Acetoxy-2-naphthoesäure (Reinheit: 90%, HPLC) (75%, bezogen auf 6-Hydroxy-2-isopropylnaphthalin).

Durch Umkristallisieren aus Wasser, Wasser/Alkoholgemisch oder verdünnter Essigsäure kann die erhaltene 6-Acetoxy-2-naphthoesäure weitere gereinigt werden.

Durch Hydrolyse mit wässriger Natriumhydroxidlösung kann sie in 6-Hydroxy-2-naphthoesäure umgewandelt werden.

Analog kann man auch 6-Hydroxy-2-methylnaphthalin zu 6-Hydroxy-2-naphthoesäure oxidieren.

Vergleichsbeispiel 1

7,8 g 6-Acetoxy-2-isopropylnaphthalin werden in 40 ml Essigsäure gelöst und in die Reaktionsapparatur wie in Beispiel 1 gegeben. Die Lösung wird auf 110°C erhitzt; dann beginnt man mit der Sauerstoffzufuhr (80 ml/min) und gibt in dieser Reihenfolge 0,2 g Di-tert.-butyl-peroxid, 0,21 g Kobalt(II)acetat-tetrahydrat und 0,24 g Mangan(II)bromid-tetrahydrat unter Rühren in die Reaktionslösung.

Nach einer Reaktionsdauer von 6 Stunden sind nur etwa 30% des Ausgangsproduktes umgesetzt.

Vergleichsbeispiel 2

5 g 6-Hydroxy-2-isopropylnaphthalin werden in 40 g Essigsäure gelöst und in die Reaktionsapparatur wie im Beispiel 1 gegeben; dann beginnt man mit der Sauerstoffzufuhr und gibt in dieser Reihenfolge und unter Rühren 0,2 g Di-tert.-butyl-peroxid, 0,17 g Kobalt(II)acetat-tetrahydrat und 0,2 g Mangen(II)bromid-tetrahydrat in die Reaktionslösung.

In der Reaktionslösung können nach 6 Stunden Reaktionsdauer keine Oxidationsprodukte nachgewiesen werden (HPLC). Das eingesetzt 6-Hydroxy-2-isopropylnaphthalin wird unter den angegebenen Bedingungen nicht oxidiert.

Beispiel 2

5 g 4-Methylphenol (Reinheit: 99%, HPLC), gelöst in 40 g Essigsäureanhydrid, werden in einem 100 ml-Vierhalskolben, versehen mit Rührer, Thermometer, Rückflusskühler und Gaseinlassfritte gegeben, unter Rühren mit 3 Tropfen Bromwasserstoffsäure (48%) versetzt und dann auf 110°C erhitzt. Nach 30 Minuten Reaktionszeit bei dieser Temperatur beginnt man mit der Sauerstoffzufuhr in einer Menge von 80 ml/min und gibt gleichzeitig 0,25 g Kobalt(II)acetat-tetrahydrat und 0,29 g Mangan(II)bromid-tetrahydrat in die Reaktionslösung. Zu dieser Zeit beträgt das Gewichtsverhältnis von Essigsäure zu Essigsäureanhydrid etwa 1 : 4.

Um die Initiierung der Oxidation zu beschleunigen, kann man auch einen sogenannten Initiator, z.B. 0,1 g Di-tert.-butylperoxid hinzugeben.

Die Reaktion wird nach 6 Stunden durch Abstellen der Sauerstoffzufuhr beendet. Das Gewichtsverhältnis von Essigsäure zu Essigsäureanhydrid steigt während der Reaktion auf 1 : 1. Das Gewichtsverhältnis der Verbindung der Formel (8) zur Verbindung der Formel (7) (R = $CH_3$) beträgt 1,6 : 1. Das Reaktionsgemisch wird auf Raumtemperatur abgekühlt. Die dabei ausfallende 4-Acetoxybenzoesäure wird abgetrennt, mit Wasser gewaschen und getrocknet. Ausbeute: 4,1 g (50%), Reinheit: 99% (HPLC). Das mit den Reaktionsprodukten der Formeln (7) und (8) bei Raumtemperatur gesättigte Filtrat kann als Reaktionsmedium wiederverwendet werden (Beispiel 3).

Beispiel 3

5 g 4-Methylphenol, gelöst in 10 g Essigsäureanhydrid, werden wie im Beispiel 2 beschrieben acetyliert. Das Reaktionsgemisch wird mit dem bei der Reaktion gemäss Beispiel 2 anfallenden Filtrat vereinigt. Man erwärmt das Gemisch auf 110°C, gibt 0,1 g Di-tert.-butyl-peroxid hinzu und beginnt mit der Sauerstoffzufuhr in einer Menge von 80 ml/min. Nach 6 Stunden wird die Reaktion durch Abstellen der Sauerstoffzufuhr beendet und das Reaktionsgemisch auf Raumtemperatur abgekühlt. Dabei fällt 8,6 g 4-Acetoxybenzoesäure aus. Vor dem Abkühlen werden folgende Gewichtsverhältnisse im Reaktionsgemisch bestimmt: Essigsäure zu Essigsäureanhydrid = 3 : 1 und Verbindung der Formel 8 zur Verbindung der Formel (7) = 6,1 : 1. Ausbeute: 8,6 g, Reinheit: 98% (HPLC). Die Ausbeute liegt über 100% der Theorie (bei etwa 106%), da aus dem Filtrat gemäss Beispiel 2, das hier verwendet wurde, ein zusätzlicher Teil 4-Acetoxybenzoesäure ausfällt (zurückzuführen auf das grössere Verhältnis von Essigsäure zu Essigsäureanhydrid).

Die Reaktion kann auch ohne die Zugabe von 0,1 g Di-tert.-butylperoxid durchgeführt werden.

Beispiel 4

5 g 4-Methylphenol werden wie in Beispiel 2 beschrieben acetyliert, jedoch nur mit 10 g Essigsäurenahydrid und während einer Reaktionszeit von 1,5 Stunden. Nach der Acetylierung ergibt sich ein Essigsäure/Essigsäureanhydrid-Gewichtsverhältnis von 1,4 : 1. Ist die Acetylierung beendet, fügt man 30 g Essigsäure hinzu und

führt die Oxidationsreaktion wie im Beispiel 2 durch. Nach 6 Stunden Reaktionszeit kann im Reaktionsgemisch keine nennenswerte Menge an Essigsäureanhydrid mehr festgestellt werden. Nach beendeter Reaktion wird das Gemisch auf Raumtemperatur abgekühlt und die ausgefallene 4-Acetoxybenzoesäure abgetrennt, mit Wasser gewaschen und anschliessend getrocknet. Ausbeute: 4,7 g (57%). Reinheit: 99% (HPLC).

Das mit dem Reaktionsprodukt gesättigte Filtrat wird im Beispiel 5 wiederverwendet.

Beispiel 5

5 g 4-Methylphenol werden wie im Beispiel 4 beschrieben acetyliert. Das resultierende Reaktionsgemisch wird mit dem bei der Reaktion gemäss Beispiel 4 anfallenden Filtrat vereinigt. Man erwärmt das Gemisch auf 110°C, gibt 0,1 g Di-tert.-butyl-peroxid hinzu und beginnt mit der Sauerstoffzufuhr in einer Menge von 80 m/min. Nach 3 Stunden kann noch keine Oxidation festgestellt werden. Erst nach Zugabe von zusätzlichen 5 g Essigsäureanhydrid beginnt die Reaktion. Nach weiteren 6 Stunden kann durch Abkühlen des Reaktionsgemisches auf Raumtemperatur 7,1 g 4-Acetoxybenzoesäure durch Auskristallisieren gewonnen werden. Ausbeute: 7,1 g (86%), Reinheit: 99% (HPLC).

**Patentansprüche**

1. Verfahren zur Herstellung von Verbindungen der Formel

(1) HO - Ar - COOH ,

worin Ar ein gegebenenfalls substituiertes aromatisches Ringsystem, vorzugsweise gegebenenfalls substituiertes Phenylen oder Naphthylen ist, dadurch gekennzeichnet, dass man Verbindungen der Formel

(2) RO - Ar - $R_1$ ,

worin R Wasserstoff oder $R_2CO$-, worin $R_2$ Wasserstoff oder $C_1$-$C_4$Alkyl, $R_1$ Alkyl mit 1 bis 5 Kohlenstoffatomen ist und Ar die angegebene Bedeutung hat, in einem organischen Lösungsmittel bei 80 bis 130°C und in Gegenwart eines Acylierungsmittels, einer Kobalt- und/oder Manganverbindung und einer Bromidionen liefernden Verbindung mit Sauerstoff oder Sauerstoff abgebenden Verbindungen oxidiert und anschliessend hydrolysiert.

2. Verfahren nach Anspruch 1, dadurch gekennzeichnet, dass die Verbindungen der Formel (2) den Formeln

(3) 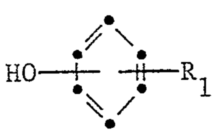 und (4) 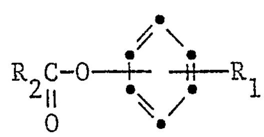 oder

(5) 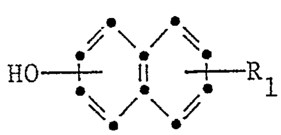 und (6) 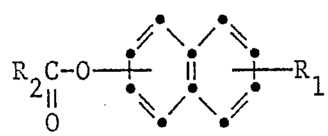

entsprechen, worin $R_1$ die in Anspruch 1 angegebene Bedeutung hat und $R_2$ Wasserstoff, $C_1$-$C_4$-Alkyl, insbesondere $C_1$-$C_3$-Alkyl oder Phenyl ist.

3. Verfahren nach den Ansprüchen 1 oder 2, dadurch gekennzeichnet, dass die Verbindungen der Formel (2) den Formeln

(3) 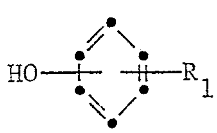 oder (5) 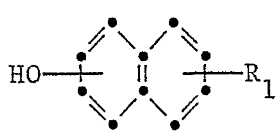

entsprechen, worin $R_1$ die in Anspruch 1 angegebene Bedeutung hat.

4. Verfahren nach Anspruch 3, dadurch gekennzeichnet, dass die Verbindungen der Formel (3) p-Methyl- oder p-Isopropylphenol und die Verbindungen der Formel (5) 2-Methyl- oder 2-Isopropyl-6-hydroxynaphthalin sind.

5. Verfahren nach mindestens einem der Ansprüche 1 bis 4, dadurch gekennzeichnet, dass die organischen Lösungsmittel niedermolekulare Alkylcarbonsäuren mit 1 bis 5, vorzugsweise 2 bis 4 Kohlenstoffatomen sind.

6. Verfahren nach mindestens einem der Ansprüche 1 bis 5, dadurch gekennzeichnet, dass die Acylierungsmittel Anhydride von niedermolekularen Alkylcarbonsäuren mit 1 bis 5, vorzugsweise 2 bis 4 Kohlenstoffatomen sind.

7. Verfahren nach mindestens einem der Ansprüche 1 bis 6, dadurch gekennzeichnet, dass man als Oxidationskatalysatoren Oxide, Hydroxide sowie anorganische oder organische Salze des Kobalts oder Mangans und deren Mischungen und als Bromidionen liefernde Verbindungen Brom, Bromwasserstoffsäure sowie anorganische Bromide oder organische Bromverbindungen verwendet.

8. Verfahren nach Anspruch 7, dadurch gekennzeichnet, dass man als Oxidationskatalysatoren die Salze des Kobalts oder Mangans in Mengen von jeweils 2 bis 4 Mol-%, bezogen auf 1 Mol der Verbindungen der Formel (2) einsetzt.

9. Verfahren nach Anspruch 7, dadurch gekennzeichnet, dass man als Oxidationskatalysatoren Gemische der Salze des Kobalts und Mangans in Mengen von 4 bis 8 Mol-%, bezogen auf 1 Mol der Verbindungen der Formel (2) einsetzt.

10. Verfahren nach mindestens einem der Ansprüche 1 bis 9, dadurch gekennzeichnet, dass man die Oxidation in Gegenwart eines zusätzlichen radikalbildenden Reaktionsinitiators durchführt.

11. Verfahren nach Anspruch 10, dadurch gekennzeichnet, dass man organische Peroxid- oder Azoverbindungen als Reaktionsinitiatoren verwendet.

12. Verfahren nach Anspruch 1, dadurch gekennzeichnet, dass man die Hydrolyse mit Mineralsäuren, vorzugsweise Salzsäure, oder Alkalimetallhydroxiden, vorzugsweise Natriumhydroxid, in üblicher Weise durchführt.

13. Verfahren nach mindestens einem der Ansprüche 1 bis 12, dadurch gekennzeichnet, dass man die Oxidation bei Temperaturen von 90 bis 130° C, insbesondere bei 110 bis 125° C durchführt.

14. Verfahren nach mindestens einem der Ansprüche 1 bis 13, dadurch gekennzeichnet, dass man die Oxidation kontinuierlich durchführt.

15. Die nach dem Verfahren nach mindestens einem der Ansprüche 1 bis 14 hergestellten Verbindungen der Formel (1), insbesondere 4-Hydroxybenzoesäure oder 6-Hydroxy-2-naphthoesäure.

16. Verwendung der Verbindungen nach Anspruch 15 als Zwischenprodukte zur Herstellung von Farbstoffen, Kunststoffen oder Pharmazeutika.